# EUROPEAN PATENT APPLICATION

(11) **EP 4 800 106 A1**
(43) Date of publication of application: **02.09.2026**
(21) Application number: 24881699.3
(22) Date of filing: 24.10.2024
(51) Int. Cl.: C12N 9/50, G01N 33/78, G01N 21/64, G01N 33/558

(54) **DISSOCIATION AGENT AND KIT**

(30) Priority: 26.10.2023 CN 202311400797
(71) Applicant: Leadway (HK) Limited, Sheung Wan, Hong Kong (CN)
(72) Inventor: WU, Wei, Hangzhou, Zhejiang 310030 (CN); CHENG, Shuyan, Hangzhou, Zhejiang 310030 (CN); YANG, Qian, Hangzhou, Zhejiang 310030 (CN); MAO, Limin, Hangzhou, Zhejiang 310030 (CN); TANG, Baojun, Hangzhou, Zhejiang 310030 (CN)
(74) Representative: Schiweck Weinzierl Koch Patentanwälte Partnerschaft mbB
(86) International application number: PCT/CN2024/127071
(87) International publication number: WO 2025/087337

(57) **Abstract**

Disclosed is a dissociation reagent and a detection kit for thyroid function testing, wherein the dissociation reagent includes a protease and a reducing agent, and is capable of effectively dissociating bound T3 and T4 in serum from binding proteins to become free forms. The dissociation reagent and the kit, when applied to fluorescence immunochromatographic detection, can effectively reduce signal interference and improve detection sensitivity and accuracy.

## Description

### TECHNICAL FIELD

This invention relates to the technical field of biological detection and, in particular, to a dissociation reagent and a kit for detecting total triiodothyronine (TT3) and/or total thyroxine (TT4) in a sample.

### BACKGROUND

Triiodothyronine (T3) and thyroxine (T4) are the main products secreted by the thyroid gland and are also essential components of the hypothalamus-anterior pituitarythyroid regulatory system. They have an impact on anabolic metabolism and are important components in clinical thyroid function tests. T3 and T4 can assist in the diagnosis of thyroid cancer. In human blood, over 99% of T3 and T4 are bound to their respective binding proteins, primarily to thyroxine-binding globulin (TBG) and, to a lesser extent, to albumin and thyroxine-binding prealbumin (TBPA). The T3 and T4 in a bound state are difficult to detect. The primary function of a dissociation reagent is to dissociate the bound T3 and T4 from their respective binding proteins, converting them into free molecules to facilitate their detection by related reagents. The degree of dissociation is closely related to the accuracy of the actual detection. Therefore, for the detection of total T3 (TT3) and total T4 (TT4) in serum, it is essential to completely dissociate the bound T3 or T4 molecules to obtain an accurate measurement of TT3 or TT4 content in serum.

In recent years, rapid immunological diagnostic technologies and detection methods have seen rapid development and are widely applied in a range of fields, including medical testing, pharmaceutical analysis, environmental analysis, food analysis, and biomedicine. With its numerous advantages such as high speed, simplicity, accuracy, and stability, immunochromatography has become one of the commonly used technologies in clinical diagnostics today. Based on the principle of immunochromatography, signal substances (hereinafter referred to as signal markers) used to label antibodies or antigens accumulate on the test line (T line) of the immunoassay strip, generating a specific signal. The magnitude of the signal correlates with the concentration of the to-be-detected analyte (antibody, antigen, or hapten), thereby enabling qualitative and/or quantitative detection of the analyte. Signal markers can be classified into different types, including colored colloidal particles (latex microspheres, colloidal gold, colloidal carbon, colloidal selenium, etc.), fluorescent microspheres, time-resolved fluorescent microspheres, colored fluorescent particles, quantum dots, up-conversion luminescent materials (such as up-conversion fluorescent materials and up-conversion phosphorescent materials), and magnetic nanoparticles.

Currently, the dissociation reagents used for TT3/TT4 chemiluminescence reagent platforms (e.g., from Abbott and Beckman), and for TT3/TT4 enzyme-linked immunoassay platforms (e.g., from ACON), primarily contain 8-Anilino-1-naphthalenesulfonic acid ammonium salt (ANS). The fluorescent immunochromatography platform, as a rapid diagnostic technology capable of quantitatively detecting analytes, relies on its fluorescent signal value for detection accuracy. ANS solution exhibits strong fluorescence, which introduces a certain degree of background interference in fluorescent immunochromatography platform detection, leading to reduced sensitivity and inaccurate results.

### SUMMARY

In view of the deficiencies in the prior art, this invention disclosure provides a novel dissociation reagent formulation that effectively dissociates bound T3 and T4 in serum from their respective binding proteins. Subsequently, the clinical experimental data of samples are obtained by measuring the content of free T3 or T4, thereby improving the sensitivity and accuracy of clinical measurements of TT3 and TT4.

This invention provides a dissociation reagent for detecting TT3 and/or TT4 in a sample, comprising a reducing agent and a protease that releases bound T3 or T4 from T3 binding proteins or T4 binding proteins.

This invention further provides a kit for detecting TT3 and/or TT4 in a sample, including a dissociation reagent, wherein the dissociation reagent includes a reducing agent and a protease that releases bound T3 or T4 from T3 binding proteins or T4 binding proteins.

In some embodiments of this invention, the protease is selected from elastase or pepsin. In some embodiments, the elastase is from porcine pancreas. In some embodiments, a concentration of the protease is 0.05-5 g/L, and a concentration of the reducing agent is 0.03-5 g/L; more specifically, the concentration of the protease is 0.1-2 g/L, and the concentration of the reducing agent is 0.1-3 g/L.

In some embodiments, the reducing agent is a disulfide bond reducing agent, commonly used for reducing disulfide bonds in proteins and dissociating T3 and T4 from their bound state by disrupting binding between T3 and T4 and their respective binding proteins. In specific embodiments, the disulfide bond reducing agent may be selected from dithiothreitol (DTT), tris(2-carboxyethyl)phosphine hydrochloride (TCEP-HCl), or a combination thereof.

In some embodiments, the dissociation reagent further includes a surfactant. In some embodiments, a concentration of the surfactant is 0.01%-5% (v/v); specifically, the concentration of the surfactant is 0.5%-2% (v/v). In some embodiments, the surfactant is a non-ionic surfactant, such as Tween-20, Tween-21, Tween-40, Tween-60, Tween-61, Tween-80, Tween-81, Tween-85, Triton X-100, or a combination thereof.

In some embodiments, the dissociation reagent further includes a buffer. In some embodiments, the pH of the buffer is 6.5-8.5; specifically, the pH of the buffer is 7.0-8.5. In some embodiments, the buffer is selected from PBS or Tris-HCl.

In some embodiments, the dissociation reagent further includes an N-heterocyclic compound. In some embodiments, a concentration of the N-heterocyclic compound is 0.02-5 g/L; specifically, the concentration of the N-heterocyclic compound is 0.1-2 g/L. In some embodiments, the N-heterocyclic compound is selected from 5-bromo-2-methylpyridine, 3-bromo-5-hydroxypyridine, or a combination thereof. The N-heterocyclic compound dissociates T3 and T4 by competitively binding to their respective binding proteins in biological fluids such as blood, while also preventing the dissociated T3 and T4 from re-binding to their respective binding proteins.

In some embodiments of this invention, the dissociation reagent further comprises a preservative, a concentration of the preservative is 0.01%-2%; specifically, the concentration of the preservative is 0.1%-2%. In some embodiments of this invention, the preservative is selected from Proclin-150, Proclin-200, Proclin-300, Proclin-5000, sodium azide, thimerosal, sodium benzoate, or a combination thereof.

In some embodiments, the kit further comprises a conjugate of a thyroid hormone or an analog thereof with a carrier protein and a thyroid hormone antibody labeled with a fluorescent marker, wherein the thyroid hormone is selected from T3, T4, or a combination thereof. In some embodiments, the conjugate of the thyroid hormone or the analog thereof with the carrier protein is coated on a test line of an immunochromatographic strip, and the thyroid hormone antibody labeled with the fluorescent marker is located upstream of the test line during detection and moves toward the test line after a sample is added.

In some embodiments of this invention, the fluorescent marker is selected from a time-resolved fluorescent molecule, a time-resolved fluorescent microsphere, a fluorescent compound, or a colored fluorescent microsphere. In some embodiments of this invention, the fluorescent marker is a time-resolved fluorescent microsphere internally encapsulated with a lanthanide element or a chelate thereof. Furthermore, the kit may further include a test card, instructions for use, and the like.

This invention disclosure further provides a method for preparing a dissociation reagent, the dissociation reagent comprising an elastase, a reducing agent, a surfactant, an N-heterocyclic compound, and a buffer, wherein during preparation, the buffer is first prepared and adjusted to pH 7.1-7.4; subsequently, the elastase, the reducing agent, and the N-heterocyclic compound are added and stirred to mix uniformly; and after complete mixing and dissolution, the surfactant is added and stirred to mix uniformly. Additionally, this invention has also found that when the pH is adjusted to 7.0-8.5, TT3 and TT4 in the sample can also be accurately detected.

Beneficial effects: (1) this invention provides a novel dissociation reagent formulation capable of relatively fully dissociating bound T3 and T4 in clinical samples from their respective binding proteins; (2) this invention found that the prior art dissociation reagent containing ANS, when used in fluorescence chromatography platforms, interferes with the fluorescent signal generated by the fluorescent marker on the test line, resulting in high background fluorescence noise. This leads to abnormal fluorescent signal values in sample detection and introduces significant deviations in overall detection concentration. Employing the novel dissociation reagent formulation provided by this invention does not interfere with the fluorescent signal generated by the fluorescent marker on the test line, and the background fluorescence noise is relatively low. Consequently, it enhances the sensitivity and accuracy of TT3 and TT4 detection on fluorescence immunochromatography platforms, and demonstrates favorable clinical correlation with detection values of Abbott chemiluminescence reagents.

### BRIEF DESCRIPTION OF THE DRAWINGS

FIG. 1 is an exploded view of a test card used in the invention.
FIG. 2 is a schematic view of an immunoassay strip in the test card used in the invention.

### DETAILED DESCRIPTION OF THE EMBODIMENTS

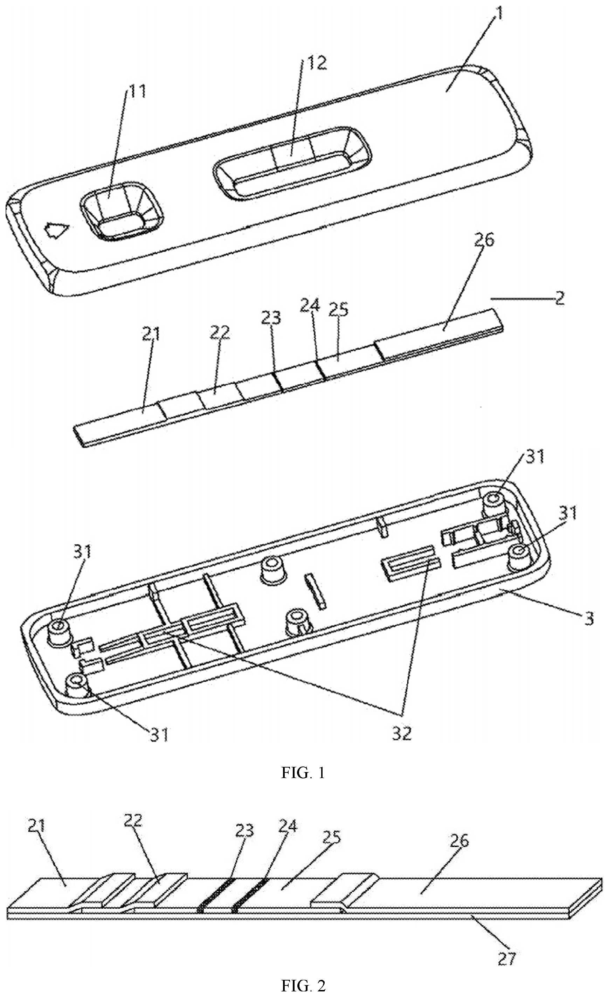

As shown in FIGs. 1 and 2, a test card includes a card cover 1, an immunoassay strip 2, and a card base 3. The immunoassay strip 2 used is an immunochromatographic strip, which includes a sample pad 21, a conjugate pad 22, a detection pad 25, and an absorbent pad 26 that are overlapped in sequence. The detection pad 25 is made from materials such as nitrocellulose, glass fiber, polyethersulfone, or nylon; for example, the detection pad 25 is a nitrocellulose membrane. A test line 23 and a control line 24 are arranged on the detection pad 25. The sample pad 21 is made of a water-absorbent material, which may be selected from glass fiber or non-woven fabric. The conjugate pad 22 is also made of a water-absorbent material, which may be selected from a polyester membrane, glass fiber, or non-woven fabric.

The immunoassay strip 2 further includes a backing layer 27, and the backing layer 27 is made of a common hydrophobic material such as polyvinyl chloride, ensuring that samples cannot leak through the backing layer 27. The detection pad 25 is disposed on the backing layer 27. The sample pad 21 is disposed on the backing layer 27, with one end of the sample pad 21 partially overlapping the conjugate pad 22; the conjugate pad 22 is disposed on the backing layer 27, with one end of the conjugate pad 22 partially overlapping the sample pad 21 and the other end of the conjugate pad 22 partially overlapping the detection pad 25; the absorbent pad 26 is disposed on the backing layer 27, made of a hydrophilic material, which may be selected from filter paper; and one end of the absorbent pad 26 partially overlaps the detection pad 25. Furthermore, in some cases, the overlapping region between any two adjacent pads has a length of 0.5-5 mm.

The immunoassay strip 2 is located within a housing, which is formed by combining the card cover 1 and the card base 3 through methods such as ultrasonic welding, snap-fit connection, or adhesive bonding. In some cases, the card cover 1 and the card base 3 are optionally made of plastic material. The card base 3 is provided with a test strip slot 32 in the middle for accommodating the immunoassay strip 2. In some cases, the card cover 1 is provided with a plurality of downward extending snap-fit fasteners (not shown), and the card base 3 is provided with a plurality of upwardly extending snap-fit slots 31, wherein the snap-fit fasteners on the card cover 1 and the snap-fit slots 31 on the card base 3 correspond one-to-one, so that when the card cover 1, the immunoassay strip 2, and the card base 3 are assembled together, the card cover 1 and the card base 3 can be securely fixed together, and the immunoassay strip 2 is secured within the test strip slot 32. In some cases, the plurality of snap-fit fasteners on the card cover 1 are symmetrically distributed on both sides of the card cover 1, and the plurality of snap-fit slots 31 on the card base 3 are symmetrically distributed on both sides of the card base 3.

The card cover 1 is further provided with a sample adding port 11 and an observation window 12. When a clinical sample is loaded through the sample adding port 11, the sample enters the sample pad 21 located below the sample adding port 11 and migrates along the length direction of the immunoassay strip 2 toward the absorbent pad 26 under capillary action. The observation window 12 is disposed above the test line 23 and the control line 24 on the detection pad 25. Excitation light emitted from an external light source 4 can pass through the transparent or semi-transparent observation window 12 to irradiate the test line 23 and the control line 24 on the immunoassay strip 2. After irradiation by the excitation light, light emitted from a fluorescent marker on the test line 23 and the control line 24 can also be output through the observation window 12 to a detector 7.

During detection, a test card is inserted into a test card insertion port of an analyzer. After a clinical sample is loaded onto the test card, the test card may be inserted into the analyzer for detection after reacting for a period of time outside the analyzer, or it may be inserted into the analyzer immediately after the clinical sample is loaded, and then detected after reaction for a period of time within the analyzer. The analyzer used in this invention may be a commercially available analyzer, such as the FIC-Q100N Dry Fluorescence Immunoassay Analyzer (Suzhou Helmen Precision Instruments Co., Ltd.).

Depending on analytes (e.g., antigens, antibodies, or haptens) and the principle of immunoassay, substances coated on the conjugate pad 22 and the test line may vary. For example, taking analyte as TT3 and the detection principle as a competitive assay as an example, the conjugate pad 22 is coated with a T3 antibody labeled with a fluorescent marker and a rabbit IgG antibody labeled with the fluorescent marker; the test line 23 is coated with a conjugate of T3 antigen and a carrier protein BSA; and the control line 24 is coated with a goat anti-rabbit IgG antibody. Thus, when a clinical sample pre-treated with the dissociation reagent is loaded onto the sample pad 21 through the sample adding port 11, the clinical sample carries the T3 antibody labeled with the fluorescent marker and the rabbit IgG antibody labeled with the fluorescent marker, flowing along the length direction of the immunoassay strip 2. When the clinical sample reaches the conjugate pad 22, the T3 antibody labeled with the fluorescent marker specifically binds to T3 (if present) in the clinical sample. The formed fluorescent marker-T3 antibody-T3 complex continues to flow. The T3-BSA conjugate coated on the test line 23 competes with the T3 in the clinical sample for the T3 antibody labeled with the fluorescent marker. After irradiation of the test line 23 by the excitation light from the light source in the analyzer, a detection signal can be generated. When flowing to the control line 24, the rabbit IgG antibody labeled with the fluorescent marker specifically binds to the goat anti-rabbit IgG antibody coated on the control line 24, forming a complex fixed on the control line 24. After irradiation of the control line 24 by the excitation light from the light source in the analyzer, a control signal can be generated.

Given that the fluorescent signal generated by the control line in this invention may also serve only to indicate whether the loaded clinical sample has flowed to the absorbent pad 26 and does not participate in the calculation of analyte concentration, the fluorescent marker in the rabbit IgG antibody labeled with the fluorescent marker may also be replaced with colored colloidal particles such as latex microspheres, colloidal gold, colloidal carbon, or colloidal selenium.

The fluorescent marker used in this invention may be a time-resolved fluorescent marker. Time-resolved fluorescent markers possess the characteristics of delayed luminescence, meaning they can continue to emit fluorescence for a certain period of time after the excitation light from the external light source is turned off. Time-resolved fluorescent markers may exist in molecular form, referred to as time-resolved fluorescent molecules, which may be selected from lanthanide elements and chelates thereof, such as samarium (Sm(III)), dysprosium (Dy(III)), europium (Eu(III)), and terbium (Tb(III)). A suitable lanthanide chelate is N-(p-isothiocyanatobenzyl)-diethylenetriamine tetraacetic acid-Eu⁺³. Time-resolved fluorescent markers may also exist in another form: time-resolved fluorescent microspheres, wherein time-resolved fluorescent molecules are encapsulated inside or on the surface of natural or synthetic microspheres or microbeads. Each time-resolved fluorescent microsphere can encapsulate thousands of fluorescent molecules, effectively enhancing detection sensitivity. The fluorescent marker in this invention may be selected from time-resolved fluorescent microspheres with a diameter of 100-400 nm, an excitation wavelength of 360 nm, and an emission wavelength of 615 nm, which embed europium chelates internally and carry carboxyl groups on their surface (Suzhou VDO Biotech Co., Ltd., Article No.: FT0200CA).

The fluorescent marker used in this invention may also be a fluorescent compound, which, when irradiated with excitation light of an appropriate wavelength, can produce a fluorescent signal without the characteristics of delayed luminescence. It may be selected from quantum dots; fluorescein and derivatives thereof, such as fluorescein isothiocyanate (FITC); or fluorescent proteins and improved variants thereof, such as green fluorescent protein, red fluorescent protein, blue fluorescent protein, yellow fluorescent protein, orange fluorescent protein, and the like.

The fluorescent marker used in this invention may also be a colored fluorescent microsphere, which is a microsphere or microbead that encapsulates a fluorescent compound without the characteristics of delayed luminescence on its surface or internally. When irradiated with excitation light of an appropriate wavelength, it can produce a fluorescent signal without the characteristics of delayed luminescence. The colored luminescent microsphere may be selected from green fluorescent microspheres, blue fluorescent microspheres, red fluorescent microspheres, yellow fluorescent microspheres, and multicolored fluorescent microspheres (emitting fluorescence of multiple specific colors).

Whether in the time-resolved fluorescent microsphere or in the colored fluorescent microsphere, the polymer forming the microsphere or microbead may be selected from polystyrene, styrene-butadiene, styrene-acrylic-ethylene terpolymer, polymethyl methacrylate, polyethyl methacrylate, styrene-maleic anhydride copolymer, polyvinyl acetate, polyvinylpyridine, polydivinylbenzene, polybutylene terephthalate, acrylonitrile, vinyl chloride-acrylate, and the like, or aldehyde, carboxyl, amino, hydroxyl, or hydrazide derivatives thereof, or mixtures thereof. Furthermore, the surface of the microsphere or microbead typically carries groups such as hydroxyl, carboxyl, amino, aldehyde, or sulfo groups, which can be coupled with antibody- or antigen- or hapten-carrier protein conjugates using conventional chemical coupling reagents. In some cases, the particle size of the time-resolved fluorescent microsphere is 20nm-100 µm; and the particle size of the colored fluorescent microsphere is 100nm-100 µm.

This invention will be further described in conjunction with specific examples. These examples are not intended to limit the scope of this invention but to provide a further understanding thereof.

### Example 1: Dissociation Reagent Formulations

Dissociation reagent formulation: 0.1-2 g/L of elastase; 0.1-3 g/L of reducing agent; 0.1-2 g/L of N-heterocyclic compound; 0.5%-2% (v/v) of surfactant; and buffer with pH 7.1-7.4.

Formulation 1: 0.1 g of elastase, 1.0 g of DTT, 2.0 g of 5-bromo-2-methylpyridine, 0.5% (v/v) of Tween-40, and 10 mM PBS buffer (pH 7.1-7.4) mixed in 1 L of deionized water.

Formulation 2: 1.0 g of elastase, 3.0 g of DTT, 0.1 g of 5-bromo-2-methylpyridine, 0.5% (v/v) of Tween-40, and 10 mM PBS buffer (pH 7.1-7.4) mixed in 1 L of deionized water.

Formulation 3: 2.0 g of elastase, 0.1 g of DTT, 1.0 g of 3-bromo-5-hydroxypyridine, 2% (v/v) of Tween-40, and 10 mM PBS buffer (pH 7.1-7.4) mixed in 1 L of deionized water.

Additionally, the DTT in the formulations may be replaced with TCEP-HCl; the surfactant in the formulations may also be selected from Tween-20, Tween-21, Tween-60, Tween-61, Tween-80, Tween-81, Tween-85, and Triton X-100; and the buffer salt in the formulations may also be selected from 50 mM Tris-HCl buffer (pH 7.1-7.4).

To maintain the stability of the dissociation reagent formulation during storage, a preservative may be added therein. The preservative may be selected from sodium azide, thimerosal, sodium benzoate, Proclin-150, Proclin-200, Proclin-300, and Proclin-5000, at an optional concentration of 0.1%-2% (v/v).

### Example 2: Preparation Method of Dissociation Reagent

A method for preparing a dissociation reagent for detecting TT3 and TT4 in human serum, including the following steps:

First, the buffer from Example 1 was prepared and adjusted to pH 7.1-7.4; subsequently, the elastase, reducing agent, N-heterocyclic compound, and preservative from Example 1 were added and stirred to mix uniformly; and after complete mixing and dissolution, the surfactant was added and stirred to mix uniformly.

### Example 3: TT3 Performance Evaluation

Clinical serum samples, totaling 30 cases (including low, medium, and high values), with sample numbers 1 to 30, were collected and calibrated using the Total Triiodothyronine Assay Kit (Chemiluminescent Microparticle Immunoassay) (National Medical Device Import Registration No. 20142405914) produced by Abbott in conjunction with the Abbott ARCHITECT i2000 Immunoassay Analyzer. For each sample, 40 µL was pipetted into 5 sample reaction tubes. The reaction tubes were labeled as A, B, C, D, and E plus the sample number, i.e., A1-A30, B1-B30, C1-C30, D1-D30, and E1-E30.

To each reaction tube in Group A, 100 µL of Dissociation Reagent Formulation 1 from Example 1 was added;
To each reaction tube in Group B, 100 µL of Dissociation Reagent Formulation 2 from Example 1 was added;
To each reaction tube in Group C, 100 µL of Dissociation Reagent Formulation 3 from Example 1 was added; and
To each reaction tube in Group D, 100 µL of a control dissociation reagent formulation was added. The specific formulation is as follows:
   0.5 g of EDTA, 2.5 g of 5-bromo-2-methylpyridine, 2.5 g of 3-bromo-5-hydroxypyridine, 1 mL of Proclin-300, 5 mL of Tween-40, 10 g of ANS, and 1 g of sodium citrate mixed in 1 L of deionized water.

To each reaction tube in Group E, 100 µL of a conventional treatment solution was added. The specific formulation is as follows:
1 mL of Proclin-300, 5 mL of Tween-40, 2.9 g of disodium hydrogen phosphate, and 0.296 g of sodium dihydrogen phosphate mixed in 1 L of deionized water.

After mixing the solutions in the above reaction tubes, they were allowed to stand at room temperature for 10 min.Then, detection was performed using the Total Triiodothyronine (T3) Quantitative Detection Kit (Fluorescence Immunoassay) reagent (Article No.: F131-20211) produced by ACON Biotech (Hangzhou) Co., Ltd. in conjunction with the Dry Fluorescence Immunoassay Analyzer (FIC-Q100N) produced by Suzhou Helmen Precision Instruments Co., Ltd. The obtained results were compared and analyzed against the calibrated results (hereinafter referred to as the Abbott Chemiluminescence Detection Values) from the Total Triiodothyronine Assay Kit (Chemiluminescent Microparticle Immunoassay) (National Medical Device Import Registration No. 20142405914) produced by Abbott in conjunction with the Abbott ARCHITECT i2000 Immunoassay Analyzer. The results are shown in Table 1.

**Table 1 Comparative Experimental Results of TT3 (Unit: nmol/L)**

| Sample No. | Abbott Chemiluminescence Detection Value | Formulation 1 | Formulation 2 | Formulation 3 | Control Dissociation Reagent | Conventional Treatment Solution |
|---|---|---|---|---|---|---|
| 1 | 0.32 | 0.31 | 0.29 | 0.25 | 0.53 | 0.04 |
| 2 | 0.41 | 0.38 | 0.38 | 0.36 | 0.60 | 0.07 |
| 3 | 0.52 | 0.45 | 0.43 | 0.37 | 1.44 | 0.08 |
| 4 | 0.59 | 0.49 | 0.40 | 0.36 | 0.63 | 0.14 |
| 5 | 0.65 | 0.61 | 0.57 | 0.47 | 0.46 | 0.01 |
| 6 | 0.72 | 0.59 | 0.57 | 0.52 | 0.87 | 0.24 |
| 7 | 0.82 | 0.71 | 0.64 | 0.58 | 1.25 | 0.18 |
| 8 | 0.91 | 0.85 | 0.73 | 0.71 | 1.18 | 0.61 |
| 9 | 1.05 | 1.32 | 1.25 | 1.17 | 1.57 | 1.23 |
| 10 | 1.25 | 1.20 | 1.10 | 1.08 | 1.47 | 0.88 |
| 11 | 1.42 | 1.17 | 0.98 | 0.91 | 2.81 | 0.85 |
| 12 | 1.85 | 2.63 | 2.48 | 2.07 | 1.72 | 0.12 |
| 13 | 2.03 | 1.97 | 1.74 | 1.51 | 1.02 | 0.07 |
| 14 | 2.29 | 2.99 | 1.96 | 1.64 | 1.01 | 0.12 |
| 15 | 2.68 | 3.16 | 2.55 | 2.39 | 3.10 | 0.39 |
| 16 | 2.99 | 2.85 | 2.77 | 2.63 | 2.22 | 0.47 |
| 17 | 3.14 | 2.59 | 2.34 | 2.15 | 1.08 | 0.10 |
| 18 | 3.28 | 4.03 | 3.47 | 3.38 | 3.00 | 1.20 |
| 19 | 3.68 | 3.00 | 2.55 | 2.10 | 1.18 | 0.50 |
| 20 | 3.91 | 3.32 | 2.93 | 2.62 | 1.35 | 1.36 |
| 21 | 4.15 | 4.24 | 3.94 | 4.29 | 1.66 | 2.63 |
| 22 | 4.96 | 5.20 | 5.31 | 5.36 | 1.92 | 1.57 |
| 23 | 5.91 | 5.94 | 6.36 | 6.52 | 2.16 | 1.16 |
| 24 | 6.58 | 5.93 | 6.37 | 6.38 | 1.67 | 0.95 |
| 25 | 7.51 | 6.38 | 6.29 | 6.45 | 2.87 | 1.11 |
| 26 | 7.95 | 7.25 | 6.81 | 7.38 | 1.84 | 1.83 |
| 27 | 8.23 | 6.68 | 6.97 | 6.64 | 2.05 | 1.25 |
| 28 | 8.85 | 8.53 | 7.83 | 7.32 | 3.75 | 1.62 |
| 29 | 9.12 | 7.88 | 8.01 | 7.68 | 3.92 | 0.39 |
| 30 | 9.84 | 9.11 | 9.45 | 9.26 | 4.40 | 1.35 |
| Correlation r | | 0.9873 | 0.9880 | 0.9825 | 0.7304 | 0.5888 |

From the analysis of the comparative results in Table 1, the following conclusions can be drawn:
1. After treating clinical serum samples with the conventional treatment solution (Group E), the measured values are generally significantly lower. This is because most of the TT3 in the clinical samples was not dissociated and thus could not be bound by the paired antibodies. For example, the Abbott chemiluminescence reagent detection value is 9.84 nmol/L for Sample No. 30, 9.11 nmol/L for Group A, 9.45 nmol/L for Group B, 9.26 nmol/L for Group C, but only 1.35 nmol/L for Group E.
2. After treating clinical samples with the treatment solution containing the dissociation reagent ANS (Group D), the detection values of the clinical samples are generally higher than those of clinical samples treated with the conventional treatment solution (Group E). However, since ANS itself interferes with the fluorescence signal value in the fluorescence chromatography platform, resulting in high background fluorescence noise, the fluorescence signal values for clinical sample detection are abnormal, leading to significant deviations in the overall detection concentration. In terms of clinical correlation with the Abbott chemiluminescence reagent detection values, the correlations are 0.9873, 0.9880, and 0.9825, respectively for Groups A-C, but only 0.7304 for Group D.
3. The detection values for clinical samples treated with the dissociation reagents from Groups A-C show good correlation with the Abbott chemiluminescence reagent detection values, all exceeding 0.95. This is because the dissociation reagents prepared in Groups A-C dissociate TT3 in clinical samples relatively completely, and the dissociation reagents themselves do not affect the detection of fluorescence signal values.

### Example 4: TT4 Performance Evaluation

Clinical serum samples, totaling 30 cases (including low, medium, and high values), with sample numbers 1 to 30, were collected and calibrated using the Total Thyroxine Assay Kit (Chemiluminescent Microparticle Immunoassay) (National Medical Device Import Registration No. 20162404093) produced by Abbott in conjunction with the ARCHITECT i2000 instrument. For each sample, 40 µL was pipetted into 5 sample reaction tubes. The reaction tubes were labeled as A, B, C, D, and E plus the sample number, i.e., A1-A30, B1-B30, C1-C30, D1-D30, and E1-E30.

To each reaction tube in Group A, 100 µL of Dissociation Reagent Formulation 1 from Example 1 was added;
To each reaction tube in Group B, 100 µL of Dissociation Reagent Formulation 2 from Example 1 was added;
To each reaction tube in Group C, 100 µL of Dissociation Reagent Formulation 3 from Example 1 was added; and
To each reaction tube in Group D, 100 µL of a control dissociation reagent formulation was added. The specific formulation is as follows:
   0.5 g of EDTA, 2.5 g of 5-bromo-2-methylpyridine, 2.5 g of 3-bromo-5-hydroxypyridine, 1 mL of Proclin-300, 5 mL of Tween-40, 10 g of ANS, and 1 g of sodium citrate mixed in 1 L of deionized water.

To each reaction tube in Group E, 100 µL of a conventional treatment solution was added. The specific formulation is as follows:
1 mL of Proclin-300, 5 mL of Tween-40, 2.9 g of disodium hydrogen phosphate, and 0.296 g of sodium dihydrogen phosphate mixed in 1 L of deionized water.

After mixing the solutions in the above reaction tubes, they were allowed to stand at room temperature for 10 min; then, detection was performed using the Total Thyroxine (T4) Quantitative Detection Kit (Fluorescence Immunoassay) reagent registered by ACON Biotech (Hangzhou) Co., Ltd. (Article No.: F131-202311) in conjunction with the Dry Fluorescence Immunoassay Analyzer (FIC-Q100N) produced by Suzhou Helmen Precision Instruments Co., Ltd. The obtained results were compared and analyzed against the calibrated results (hereinafter referred to as the Abbott Chemiluminescence Detection Values) from the Total Thyroxine Assay Kit (Chemiluminescent Microparticle Immunoassay) (National Medical Device Import Registration No. 20162404093) produced by Abbott in conjunction with the Abbott ARCHITECT i2000 Immunoassay Analyzer. The results are shown in Table 2.

**Table 2 Comparative Experimental Results of TT4 (Unit: nmol/L)**

| Sample No. | Abbott Chemiluminescence Detection Value | Formulation 1 | Formulation 2 | Formulation 3 | Control Dissociation Reagent | Conventional Treatment Solution |
|---|---|---|---|---|---|---|
| 1 | 5.12 | 4.16 | 3.96 | 3.48 | 10.87 | 0.91 |
| 2 | 7.37 | 6.96 | 5.94 | 6.20 | 12.01 | 1.06 |
| 3 | 10.42 | 8.77 | 7.09 | 8.12 | 11.47 | 0.08 |
| 4 | 15.87 | 13.52 | 11.57 | 12.07 | 10.00 | 2.14 |
| 5 | 18.47 | 16.93 | 16.34 | 16.59 | 25.75 | 0.01 |
| 6 | 21.62 | 20.32 | 18.12 | 18.38 | 25.92 | 0.20 |
| 7 | 25.87 | 33.27 | 28.99 | 29.76 | 19.29 | 0.18 |
| 8 | 34.26 | 31.18 | 25.08 | 29.49 | 37.20 | 0.50 |
| 9 | 42.37 | 41.09 | 37.94 | 36.76 | 15.21 | 1.19 |
| 10 | 52.09 | 30.58 | 30.22 | 27.58 | 16.76 | 0.77 |
| 11 | 55.87 | 49.92 | 41.05 | 48.43 | 20.01 | 0.82 |
| 12 | 61.47 | 47.21 | 43.99 | 38.61 | 17.19 | 0.10 |
| 13 | 66.62 | 70.84 | 56.94 | 70.46 | 29.95 | 0.06 |
| 14 | 75.87 | 60.98 | 59.16 | 52.01 | 17.74 | 0.10 |
| 15 | 84.26 | 76.55 | 64.66 | 63.40 | 25.39 | 0.39 |
| 16 | 92.37 | 81.96 | 77.08 | 68.46 | 13.64 | 0.47 |
| 17 | 102.09 | 116.62 | 96.99 | 104.41 | 38.27 | 0.10 |
| 18 | 115.34 | 102.34 | 86.43 | 93.88 | 16.83 | 8.12 |
| 19 | 128.09 | 98.97 | 84.28 | 84.62 | 22.24 | 0.42 |
| 20 | 132.76 | 158.01 | 156.99 | 146.27 | 17.26 | 1.16 |
| 21 | 148.16 | 110.92 | 106.46 | 120.44 | 28.78 | 2.45 |
| 22 | 154.85 | 166.68 | 157.59 | 179.17 | 84.97 | 1.45 |
| 23 | 166.06 | 163.09 | 164.83 | 169.18 | 76.40 | 1.12 |
| 24 | 176.02 | 177.95 | 166.80 | 191.12 | 42.93 | 1.02 |
| 25 | 182.27 | 185.86 | 198.53 | 204.26 | 15.16 | 1.01 |
| 26 | 196.85 | 215.00 | 195.65 | 231.70 | 48.74 | 1.72 |
| 27 | 207.52 | 249.39 | 227.88 | 237.62 | 26.87 | 10.37 |
| 28 | 227.44 | 213.57 | 194.76 | 209.17 | 91.09 | 11.62 |
| 29 | 254.15 | 230.13 | 222.23 | 241.87 | 80.22 | 40.39 |
| 30 | 288.73 | 299.43 | 290.18 | 278.14 | 78.71 | 31.38 |
| Correlation r | | 0.9819 | 0.9797 | 0.9778 | 0.7796 | 0.7255 |

From the analysis of the comparative results in Table 2, the following conclusions can be drawn:
1. After treating clinical serum samples with the conventional treatment solution (Group E), the measured values are generally significantly lower. This is because most of the TT4 in the clinical samples was not dissociated and thus could not be bound by the paired antibodies. For example, for Sample No. 26, the Abbott chemiluminescence reagent detection value is 196.82 nmol/L, while the values for Groups A, B, C, and E were 215.00, 195.65, 231.70, and 1.72 nmol/L, respectively.
2. After treating samples with the treatment solution containing the dissociation reagent ANS (Group D), the detection values of the clinical samples are generally higher than those of clinical samples treated with the conventional treatment solution (Group E). However, since ANS itself interferes with the fluorescence signal value in the fluorescence chromatography platform, resulting in high background fluorescence noise, the fluorescence signal values for sample detection are abnormal, leading to significant deviations in the overall detection concentration. In terms of clinical correlation with the Abbott chemiluminescence reagent detection values, the correlations are 0.9819, 0.9797, and 0.9778, respectively for Groups A-C, but only 0.7796 for Group D.
3. After treating clinical samples with the treatment solution containing the dissociation reagent ANS (Group D), the detection values of the clinical samples are generally higher than those of clinical samples treated with the conventional treatment solution (Group E). However, since ANS itself interferes with the fluorescence signal value in the fluorescence chromatography platform, resulting in high background fluorescence noise, the fluorescence signal values for clinical sample detection are abnormal, leading to significant deviations in the overall detection concentration. In terms of clinical correlation with the Abbott chemiluminescence reagent detection values, the correlations are 0.9873, 0.9880, and 0.9825, respectively for Groups A-C, but only 0.7304 for Group D.
3. The detection values for clinical samples treated with the dissociation reagents from Groups A-C show good correlation with the Abbott chemiluminescence reagent detection values, all exceeding 0.95. This is because the dissociation reagents prepared in Groups A-C dissociate TT4 in clinical samples relatively completely, and the dissociation reagents themselves do not affect the detection of fluorescence signal values.

### Example 5: Reducing Agent Evaluation Experiment

Formulation 4: 0.1 g of elastase, 1.0 g of TCEP-HCl, 2.0 g of 5-bromo-2-methylpyridine, 0.5% (v/v) of Tween-40, and 10 mM PBS buffer (pH 7.1-7.4) mixed in 1 L of deionized water.

Formulation 5: 0.1 g of elastase, 3.0 g of TCEP-HCl, 2.0 g of 5-bromo-2-methylpyridine, 0.5% (v/v) of Tween-40, and 10 mM PBS buffer (pH 7.1-7.4) mixed in 1 L of deionized water.

Fifteen fresh clinical blood samples each with uniformly distributed concentrations were collected and calibrated using the Abbott Total Triiodothyronine Assay Kit (Chemiluminescent Microparticle Immunoassay) (National Medical Device Import Registration No. 20142405914) in conjunction with the Abbott ARCHITECT i2000 Immunoassay Analyzer. Then, detection was performed according to the TT3 performance detection method described in Example 3, with the difference being that only three reaction tubes were selected. Specifically, 100 µL of Dissociation Reagent Formulation 1 from Example 1 was added to the reaction tubes in Group A, 100 µL of Dissociation Reagent Formulation 4 was added to the reaction tubes in Group B, and 100 µL of Dissociation Reagent Formulation 5 was added to the reaction tubes in Group C. The results are shown in Table 3.

**Table 3 Reducing Agent Evaluation Experiment Results (Unit: nmol/L)**

| Sample No. | Abbott Chemiluminescence Detection Value | Formulation 1 | Formulation 4 | Formulation 5 |
|---|---|---|---|---|
| 1 | 0.32 | 0.27 | 0.34 | 0.21 |
| 2 | 0.73 | 0.63 | 0.56 | 0.68 |
| 3 | 0.86 | 0.76 | 0.80 | 0.98 |
| 4 | 1.07 | 0.81 | 0.98 | 0.92 |
| 5 | 1.25 | 1.02 | 1.03 | 1.65 |
| 6 | 2.03 | 2.00 | 2.18 | 1.67 |
| 7 | 2.32 | 2.18 | 2.14 | 2.58 |
| 8 | 2.76 | 2.29 | 2.59 | 2.42 |
| 9 | 2.9 | 2.41 | 2.37 | 2.62 |
| 10 | 3.45 | 3.03 | 3.19 | 3.17 |
| 11 | 4.78 | 3.84 | 4.16 | 4.14 |
| 12 | 6.02 | 5.99 | 4.83 | 5.32 |
| 13 | 7.17 | 6.24 | 7.65 | 7.67 |
| 14 | 9.28 | 9.53 | 7.54 | 7.71 |
| 15 | 11.59 | 9.08 | 9.32 | 9.18 |
| Correlation Coefficient (r) | | 0.9848 | 0.9863 | 0.9854 |

It can be seen from Table 3 that the correlation coefficients between the concentration values calculated using fluorescence immunoassay after selecting Dissociation Reagent Formulations 1, 4, and 5, and the Abbott chemiluminescence detection values are all greater than 0.95, which indicates that Dissociation Reagent Formulations 1, 4, and 5 can sufficiently dissociate TT3 in the samples, thereby yielding relatively accurate detected TT3 values in the samples.

Fifteen fresh clinical blood samples each with uniformly distributed concentrations were collected and calibrated using the Total Thyroxine Assay Kit (Chemiluminescent Microparticle Immunoassay) (National Medical Device Import Registration No. 20162404093) produced by Abbott in conjunction with the ARCHITECT i2000 instrument. Then, detection was performed according to the TT4 performance detection method described in Example 4, with the difference being that only three reaction tubes were selected. Specifically, 100 µL of Dissociation Reagent Formulation 1 from Example 1 was added to the reaction tubes in Group A, 100 µL of Dissociation Reagent Formulation 4 was added to the reaction tubes in Group B, and 100 µL of Dissociation Reagent Formulation 5 was added to the reaction tubes in Group C. The results are shown in Table 4.

**Table 4 Reducing Agent Evaluation Experiment Results (Unit: nmol/L)**

| Sample No. | Abbott Chemiluminescence Detection Value | Formulation 1 | Formulation 4 | Formulation 5 |
|---|---|---|---|---|
| 1 | 4.79 | 3.36 | 3.97 | 4.02 |
| 2 | 16.69 | 21.24 | 16.67 | 15.27 |
| 3 | 25.03 | 28.38 | 26.16 | 23.17 |
| 4 | 32.78 | 29.53 | 29.28 | 26.69 |
| 5 | 58.08 | 52.46 | 48.59 | 55.32 |
| 6 | 68.49 | 74.30 | 57.05 | 56.31 |
| 7 | 79.41 | 66.47 | 61.97 | 64.99 |
| 8 | 82.51 | 91.84 | 84.31 | 70.75 |
| 9 | 109.49 | 98.54 | 119.09 | 108.40 |
| 10 | 145.1 | 110.35 | 129.40 | 117.89 |
| 11 | 152.87 | 122.80 | 159.93 | 132.90 |
| 12 | 186 | 196.66 | 164.35 | 149.57 |
| 13 | 201.79 | 171.02 | 208.51 | 197.66 |
| 14 | 248.36 | 233.05 | 235.33 | 243.32 |
| 15 | 288.5 | 251.98 | 251.37 | 258.69 |
| Correlation Coefficient (r) | | 0.9743 | 0.9663 | 0.9811 |

It can be seen from Table 4 that the correlation coefficients between the concentration values calculated using fluorescence immunoassay after selecting Dissociation Reagent Formulations 1, 4, and 5, and the Abbott chemiluminescence detection values are all greater than 0.95, which indicates that Dissociation Reagent Formulations 1, 4, and 5 can sufficiently dissociate TT4 in the samples, thereby yielding relatively accurate detected TT4 values in the samples.

### Example 6: Surfactant Evaluation Experiment

Formulation 6: 0.1 g of elastase, 1.0 g of DTT, 2.0 g of 5-bromo-2-methylpyridine, 0.5% (v/v) of Tween-20, and 10 mM PBS buffer (pH 7.1-7.4) mixed in 1 L of deionized water.

Formulation 7: 0.1 g of elastase, 1.0 g of DTT, 2.0 g of 5-bromo-2-methylpyridine, 0.5% (v/v) of Triton X-100, and 10 mM PBS buffer (pH 7.1-7.4) mixed in 1 L of deionized water.

Fifteen fresh clinical blood samples each with uniformly distributed concentrations were collected and calibrated using the Abbott Total Triiodothyronine Assay Kit (Chemiluminescent Microparticle Immunoassay) (National Medical Device Import Registration No. 20142405914) in conjunction with the Abbott ARCHITECT i2000 Immunoassay Analyzer. Then, detection was performed according to the TT3 performance detection method described in Example 3, with the difference being that only three reaction tubes were selected. Specifically, 100 µL of Dissociation Reagent Formulation 1 from Example 1 was added to the reaction tubes in Group A, 100 µL of Dissociation Reagent Formulation 6 was added to the reaction tubes in Group B, and 100 µL of Dissociation Reagent Formulation 7 was added to the reaction tubes in Group C. The results are shown in Table 5.

**Table 5 Surfactant Evaluation Experiment Results (Unit: nmol/L)**

| Sample No. | Abbott Chemiluminescence Detection Value | Formulation 1 | Formulation 6 | Formulation 7 |
|---|---|---|---|---|
| 1 | 0.32 | 0.27 | 0.31 | 0.25 |
| 2 | 0.73 | 0.63 | 0.87 | 0.66 |
| 3 | 0.86 | 0.76 | 1.03 | 0.94 |
| 4 | 1.07 | 0.81 | 1.04 | 0.76 |
| 3 | 1.25 | 1.02 | 1.44 | 1.24 |
| 6 | 2.03 | 2.00 | 1.79 | 2.40 |
| 7 | 2.32 | 2.18 | 2.28 | 2.11 |
| 8 | 2.76 | 2.29 | 2.51 | 1.94 |
| 9 | 2.9 | 2.41 | 2.27 | 3.37 |
| 10 | 3.45 | 3.03 | 3.64 | 3.11 |
| 11 | 4.78 | 3.84 | 4.56 | 3.37 |
| 12 | 6.02 | 5.99 | 4.93 | 6.09 |
| 13 | 7.17 | 6.24 | 7.80 | 6.24 |
| 14 | 9.28 | 9.53 | 8.61 | 7.50 |
| 15 | 11.59 | 9.08 | 13.28 | 8.51 |
| Correlation Coefficient (r) | | 0.9848 | 0.9860 | 0.9799 |

It can be seen from Table 5 that the correlation coefficients between the concentration values calculated using fluorescence immunoassay after selecting Dissociation Reagent Formulations 1, 6, and 7, and the Abbott chemiluminescence detection values are all greater than 0.95, which indicates that Dissociation Reagent Formulations 1, 6, and 7 can sufficiently dissociate TT3 in the samples, thereby yielding relatively accurate detected TT3 values in the samples.

Fifteen fresh clinical blood samples each with uniformly distributed concentrations were collected and calibrated using the Total Thyroxine Assay Kit (Chemiluminescent Microparticle Immunoassay) (National Medical Device Import Registration No. 20162404093) produced by Abbott in conjunction with the ARCHITECT i2000 instrument. Then, detection was performed according to the TT4 performance detection method described in Example 4, with the difference being that only three reaction tubes were selected. Specifically, 100 µL of Dissociation Reagent Formulation 1 from Example 1 was added to the reaction tubes in Group A, 100 µL of Dissociation Reagent Formulation 6 was added to the reaction tubes in Group B, and 100 µL of Dissociation Reagent Formulation 7 was added to the reaction tubes in Group C. The results are shown in Table 6.

**Table 6 Surfactant Evaluation Experiment Results (Unit: nmol/L)**

| Sample No. | Abbott Chemiluminescence Detection Value | Formulation 1 | Formulation 6 | Formulation 7 |
|---|---|---|---|---|
| 1 | 4.79 | 3.36 | 4.30 | 4.87 |
| 2 | 16.69 | 21.24 | 15.32 | 16.68 |
| 3 | 25.03 | 28.38 | 19.20 | 17.53 |
| 4 | 32.78 | 29.53 | 37.77 | 31.59 |
| 5 | 58.08 | 52.46 | 63.62 | 62.76 |
| 6 | 68.49 | 74.30 | 77.87 | 55.82 |
| 7 | 79.41 | 66.47 | 79.21 | 77.12 |
| 8 | 82.51 | 91.84 | 79.10 | 69.51 |
| 9 | 109.49 | 98.54 | 84.33 | 80.61 |
| 10 | 145.1 | 110.35 | 139.28 | 124.48 |
| 11 | 152.87 | 122.80 | 122.69 | 170.43 |
| 12 | 186 | 196.66 | 213.75 | 150.76 |
| 13 | 201.79 | 171.02 | 228.55 | 171.03 |
| 14 | 248.36 | 233.05 | 241.00 | 223.57 |
| 15 | 288.5 | 251.98 | 268.07 | 248.38 |
| Correlation Coefficient (r) | | 0.9743 | 0.9625 | 0.9665 |

It can be seen from Table 6 that the correlation coefficients between the concentration values calculated using fluorescence immunoassay after selecting Dissociation Reagent Formulations 1, 46, and 7, and the Abbott chemiluminescence detection values are all greater than 0.95, which indicates that Dissociation Reagent Formulations 1, 6, and 7 can sufficiently dissociate TT4 in the samples, thereby yielding relatively accurate detected TT4 values in the samples.

### Example 7: Buffer Evaluation Experiment

Formulation 8: 0.1 g of elastase, 1.0 g of DTT, 2.0 g of 5-bromo-2-methylpyridine, 1.0% (v/v) of Tween-40, and 50 mM Tris-HCl buffer (pH 7.1-7.4) mixed in 1 L of deionized water.

Fifteen fresh clinical blood samples each with uniformly distributed concentrations were collected and calibrated using the Abbott Total Triiodothyronine Assay Kit (Chemiluminescent Microparticle Immunoassay) (National Medical Device Import Registration No. 20142405914) in conjunction with the Abbott ARCHITECT i2000 Immunoassay Analyzer. Then, detection was performed according to the TT3 performance detection method described in Example 3, with the difference being that only two reaction tubes were selected. Specifically, 100 µL of Dissociation Reagent Formulation 1 from Example 1 was added to the reaction tubes in Group A, and 100 µL of Dissociation Reagent Formulation 8 was added to the reaction tubes in Group B. The results are shown in Table 7.

**Table 7 Buffer Evaluation Experiment Results (Unit: nmol/L)**

| Sample No. | Abbott Chemiluminescence Detection Value | Formulation 1 | Formulation 8 |
|---|---|---|---|
| 1 | 0.32 | 0.27 | 0.31 |
| 2 | 0.73 | 0.63 | 0.70 |
| 3 | 0.86 | 0.76 | 0.66 |
| 4 | 1.07 | 0.81 | 1.06 |
| 5 | 1.25 | 1.02 | 1.14 |
| 6 | 2.03 | 2.00 | 1.45 |
| 7 | 2.32 | 2.18 | 2.22 |
| 8 | 2.76 | 2.29 | 2.68 |
| 9 | 2.9 | 2.41 | 2.39 |
| 10 | 3.45 | 3.03 | 2.79 |
| 11 | 4.78 | 3.84 | 4.45 |
| 12 | 6.02 | 5.99 | 4.90 |
| 13 | 7.17 | 6.24 | 7.02 |
| 14 | 9.28 | 9.53 | 7.08 |
| 15 | 11.59 | 9.08 | 8.31 |
| Correlation Coefficient (r) | | 0.9848 | 0.9838 |

It can be seen from Table 7 that the correlation coefficients between the concentration values calculated using fluorescence immunoassay after selecting Dissociation Reagent Formulations 1 and 8, and the Abbott chemiluminescence detection values are all greater than 0.95, which indicates that Dissociation Reagent Formulations 1 and 8 can sufficiently dissociate TT3 in the samples, thereby yielding relatively accurate detected TT3 values in the samples.

Fifteen fresh clinical blood samples each with uniformly distributed concentrations were collected and calibrated using the Total Thyroxine Assay Kit (Chemiluminescent Microparticle Immunoassay) (National Medical Device Import Registration No. 20162404093) produced by Abbott in conjunction with the ARCHITECT i2000 instrument. Then, detection was performed according to the TT4 performance detection method described in Example 4, with the difference being that only three reaction tubes were selected. Specifically, 100 µL of Dissociation Reagent Formulation 1 from Example 1 was added to the reaction tubes in Group A, and 100 µL of Dissociation Reagent Formulation 8 was added to the reaction tubes in Group B. The results are shown in Table 8.

**Table 8 Buffer Evaluation Experiment Results (Unit: nmol/L)**

| Sample No. | Abbott Chemiluminescence Detection Value | Formulation 1 | Formulation 8 |
|---|---|---|---|
| 1 | 4.79 | 3.36 | 4.65 |
| 2 | 16.69 | 21.24 | 18.73 |
| 3 | 25.03 | 28.38 | 24.71 |
| 4 | 32.78 | 29.53 | 29.88 |
| 5 | 58.08 | 52.46 | 41.67 |
| 6 | 68.49 | 74.30 | 55.32 |
| 7 | 79.41 | 66.47 | 75.42 |
| 8 | 82.51 | 91.84 | 79.84 |
| 9 | 109.49 | 98.54 | 99.73 |
| 10 | 145.10 | 110.35 | 119.81 |
| 11 | 152.87 | 122.80 | 182.13 |
| 12 | 186.00 | 196.66 | 155.97 |
| 13 | 201.79 | 171.02 | 220.39 |
| 14 | 248.36 | 233.05 | 203.81 |
| 15 | 288.50 | 251.98 | 259.86 |
| Correlation Coefficient (r) | | 0.9743 | 0.9585 |

It can be seen from Table 8 that the correlation coefficients between the concentration values calculated using fluorescence immunoassay after selecting Dissociation Reagent Formulations 1 and 8, and the Abbott chemiluminescence detection values are all greater than 0.95, which indicates that Dissociation Reagent Formulations 1 and 8 can sufficiently dissociate TT4 in the samples, thereby yielding relatively accurate detected TT4 values in the samples.

### Example 8: Dissociation Reagent Evaluation Experiment

Formulation 9: 0.1 g of elastase, 0.5% (v/v) of Tween-40, and 10 mM PBS buffer (pH 7.1-7.4) mixed in 1 L of deionized water;
Formulation 10: 1.0 g of elastase, 0.5% (v/v) of Tween-40, and 10 mM PBS buffer (pH 7.1-7.4) mixed in 1 L of deionized water;
Formulation 11: 0.1 g of elastase, 0.1 g of DTT, 0.5% (v/v) of Tween-40, and 10 mM PBS buffer (pH 7.1-7.4) mixed in 1 L of deionized water;
Formulation 12: 0.1 g of elastase, 1.0 g of DTT, 0.5% (v/v) of Tween-40, and 10 mM PBS buffer (pH 7.1-7.4) mixed in 1 L of deionized water;
Formulation 13: 1.0 g of elastase, 0.1 g of DTT, 0.5% (v/v) of Tween-40, and 10 mM PBS buffer (pH 7.1-7.4) mixed in 1 L of deionized water;
Formulation 14: 1.0 g of elastase, 1.0 g of DTT, 0.5% (v/v) of Tween-40, and 10 mM PBS buffer (pH 7.1-7.4) mixed in 1 L of deionized water.

Five fresh clinical blood samples each with uniformly distributed concentrations were collected and calibrated using the Abbott Total Triiodothyronine Assay Kit (Chemiluminescent Microparticle Immunoassay) (National Medical Device Import Registration No. 20142405914) in conjunction with the Abbott ARCHITECT i2000 Immunoassay Analyzer. Then, detection was performed according to the TT3 performance detection method described in Example 3, with the difference being that only 6 reaction tubes were selected. Specifically, 100 µL of Dissociation Reagent Formulations 9, 10, 11, 12, 13, and 14 were added to the reaction tubes in Groups A, B, C, D, E, and F, respectively, and 100 µL of the control dissociation reagent formulation from Example 3 was added to the reaction tubes in Group G. The results are shown in Table 9.

**Table 9 Dissociation Reagent Evaluation Experiment Results (Unit: nmol/L)**

| Sample No. | Abbott Chemiluminescence Detection Value | Formulation 9 | Formulation 10 | Formulation 11 | Formulation 12 | Formulation 13 | Formulation 14 | Control Dissociation Reagent |
|---|---|---|---|---|---|---|---|---|
| 1 | 0.31 | 0.51 | 0.55 | 0.42 | 0.51 | 0.42 | 0.53 | 0.62 |
| 2 | 1.06 | 0.72 | 0.67 | 0.75 | 0.68 | 0.84 | 0.79 | 0.52 |
| 3 | 2.59 | 1.52 | 1.65 | 2.14 | 1.96 | 2.21 | 2.31 | 1.65 |
| 4 | 5.98 | 3.15 | 3.25 | 3.12 | 3.15 | 3.18 | 3.24 | 2.12 |
| 5 | 8.23 | 4.98 | 5.05 | 5.69 | 6.84 | 5.51 | 5.68 | 2.13 |

It can be seen from Table 9 that, overall, compared to the control dissociation reagent formulation, Dissociation Reagent Formulations 11 to 14 can all significantly improve the accuracy of the detected TT3 values.

Five fresh clinical blood samples each with uniformly distributed concentrations were collected and calibrated using the Total Thyroxine Assay Kit (Chemiluminescent Microparticle Immunoassay) (National Medical Device Import Registration No. 20162404093) produced by Abbott in conjunction with the ARCHITECT i2000 instrument. Then, detection was performed according to the TT4 performance detection method described in Example 4, with the difference being that only 6 reaction tubes were selected. Specifically, 100 µL of Dissociation Reagent Formulations 9, 10, 11, 12, 13, and 14 were added to the reaction tubes in Groups A, B, C, D, E, and F, respectively, and 100 µL of the control dissociation reagent formulation from Example 3 was added to the reaction tubes in Group G. The results are shown in Table 10.

**Table 10 Dissociation Reagent Evaluation Experiment Results (Unit: nmol/L)**

| Sample No. | Abbott Chemiluminescence Detection Value | Formulation 9 | Formulation 10 | Formulation 11 | Formulation 12 | Formulation 13 | Formulation 14 | Control Dissociation Reagent |
|---|---|---|---|---|---|---|---|---|
| 1 | 6.98 | 8.23 | 9.13 | 8.69 | 8.96 | 8.01 | 7.98 | 12.13 |
| 2 | 20.18 | 15.69 | 12.36 | 16.98 | 16.71 | 14.36 | 13.25 | 13.25 |
| 3 | 58.64 | 32.56 | 29.14 | 40.21 | 42.65 | 36.98 | 39.18 | 32.16 |
| 4 | 104.98 | 54.18 | 50.23 | 69.14 | 71.26 | 68.24 | 62.25 | 50.65 |
| 5 | 220.15 | 140.58 | 142.39 | 165.23 | 159.23 | 150.36 | 146.12 | 109.15 |

It can be seen from Table 10 that, overall, compared to the control dissociation reagent formulation, Dissociation Reagent Formulations 11 to 14 can all significantly improve the accuracy of the detected TT4 values.

## Claims

1. A dissociation reagent, comprising a reducing agent and a protease that releases bound T3 or T4 from T3 or T4 binding proteins.

2. The dissociation reagent of claim 1, wherein a concentration of the protease is 0.05-5 g/L, preferably 0.1-2 g/L, and, preferably, the protease is selected from elastase or pepsin; a concentration of the reducing agent is 0.03-5 g/L, preferably 0.1-3 g/L, preferably, the reducing agent is a disulfide bond reducing agent, and, preferably, the disulfide bond reducing agent is selected from dithiothreitol, tris(2-carboxyethyl)phosphine hydrochloride, or a combination.

3. The dissociation reagent of claim 1, wherein the dissociation reagent further comprises a surfactant, preferably, a concentration of the surfactant is 0.01%-5% (v/v), preferably 0.5%-2% (v/v), preferably, the surfactant is a non-ionic surfactant, and, preferably, the non-ionic surfactant is selected from Tween-20, Tween-21, Tween-40, Tween-60, Tween-61, Tween-80, Tween-81, Tween-85, Triton X-100, or a combination.

4. The dissociation reagent of claim 1, wherein the dissociation reagent further comprises a buffer, a pH of the buffer is 6.5-8.5, preferably 7.0-8.5, and, preferably, the buffer is selected from PBS or Tris-HCl.

5. The dissociation reagent of claim 1, wherein the dissociation reagent further comprises an N-heterocyclic compound, preferably, a concentration of the N-heterocyclic compound is 0.02-5 g/L, preferably 0.1-2 g/L, and, preferably, the N-heterocyclic compound is selected from 5-bromo-2-methylpyridine, 3-bromo-5-hydroxypyridine, or a combination.

6. The dissociation reagent of claim 1, wherein the dissociation reagent further comprises a preservative, a concentration of the preservative is 0.01%-2%, preferably 0.1%-2%, and, preferably, the preservative is selected from Proclin-150, Proclin-200, Proclin-300, Proclin-5000, sodium azide, thimerosal, sodium benzoate, or a combination.

7. A kit, comprising the dissociation reagent of any one of claims 1-6, preferably further comprising a conjugate of a thyroid hormone or an analog thereof with a carrier protein and a thyroid hormone antibody labeled with a fluorescent marker, wherein the thyroid hormone is selected from T3, T4, or a combination.

8. The kit of claim 7, wherein the conjugate of the thyroid hormone or the analog thereof with the carrier protein is coated on a test line of an immunochromatographic strip, and the thyroid hormone antibody labeled with the fluorescent marker is located upstream of the test line during detection and moves toward the test line after a sample is added.

9. The kit of claim 7, wherein the fluorescent marker is selected from a time-resolved fluorescent molecule, a time-resolved fluorescent microsphere, a fluorescent compound, or a colored fluorescent microsphere.

10. The kit of claim 7, wherein the fluorescent marker is a time-resolved fluorescent microsphere internally encapsulated with a europium chelate.
